# EUROPEAN PATENT SPECIFICATION

(11) **EP 4 332 244 B1**
(45) Date of publication and mention of the grant of the patent: **18.11.2026**
(21) Application number: 22794467.5
(22) Date of filing: 29.03.2022
(51) Int. Cl.: C21B 13/00, C01B 3/26, C01B 3/34, C01B 3/38, C07C 11/00, B01J 23/26, B01J 23/62, C07C 5/333, C01B 3/384

(54) **IRON AND STEEL SMELTING METHOD**
EISEN- UND STAHLSCHMELZVERFAHREN
PROCÉDÉ DE FUSION DE FER ET D'ACIER

(30) Priority: 25.04.2021 CN 202110448409
(43) Date of publication of application: 06.03.2024
(73) Proprietor: China University of Petroleum-Beijing, Beijing 102249 (CN); Beijing Carbon Zero Hydrogen Electric Technology Co., Ltd, Beijing 102249 (CN)
(72) Inventor: ZHOU, Hongjun, Beijing 102249 (CN); ZHOU, Guanglin, Beijing 102249 (CN); JIANG, Weili, Beijing 102249 (CN); ZHOU, Enze, Beijing 102249 (CN); WU, Quangui, Beijing 102249 (CN)
(74) Representative: Bergenstråhle & Partners AB
(86) International application number: PCT/CN2022/083730
(87) International publication number: WO 2022/227994

(56) References cited:
- WO-A1-2018/013349
- CN-A- 101 249 949
- CN-A- 103 525 964
- CN-A- 103 525 964
- CN-A- 104 072 325
- CN-A- 107 253 895
- CN-A- 109 790 018
- CN-U- 215 517 509
- DE-A1- 102013 104 002
- US-A1- 2007 299 278

## Description

### Field of Technology

The present invention relates to a steel smelting method and belongs to the technical field of steel smelting.

### Background Art

Direct Reduced Iron (DRI), also known as sponge iron, is a kind of metallic iron obtained without blast furnace smelting, and the process of producing DRI is called non-blast furnace iron smelting process. The production process of DRI is divided into two categories: coal-based process and gas-based process. Among them, the gas-based method currently accounts for 90% of DRI production, and the typical processes are the tank method (HyL method) and the shaft furnace method (Midrex method). The shaft furnace method adopts a vertical moving bed reduction reactor, which is mainly divided into two parts: a reduction zone, where the reduction gas circulates at high temperature, hydrogen above 800°C and carbon monoxide reduce the iron oxides to produce DRI, and the hydrogen and carbon monoxide produce water and carbon dioxide; and the reduction zone located in the lower part of the reduction zone, where the hydrogen and carbon monoxide produce water and carbon dioxide; and a cooling zone located in the lower part of the reduction zone, in which the DRI in the cooling zone is cooled to ambient temperature by cooling gases containing hydrogen and carbon monoxide circulating in a cooling circuit before the DRI is discharged. Patent Literature CN 103525964 A and US 2007/299278 A1 further disclose alternative steel smelting methods.

The reductant used in the gas-based method is mainly natural gas, which is steam-converted or partially oxidized to produce a syngas CO+H₂. The price of natural gas is expensive, and the price of refined syngas produced by large-scale coal gasification is also relatively high. Thus, finding an inexpensive raw material channel for reducing gas is an issue that must be faced in order to vigorously develop DRI production.

In the olefin industry, a catalytic dehydrogenation of propane produces propylene and hydrogen, which happens to be one of the feedstocks for DRI. If it is possible to combine DRI with the olefin industry, it will revolutionize DRI.

### Summary of the invention

In order to achieve the above purpose, the present invention provides a steel smelting method that combines the olefin industry with steel smelting, utilizes the by-products of the olefin industry to provide a reducing agent for steel smelting, and realizes their efficient combination.

In order to achieve the above purpose, the present invention provides a steel smelting method as disclosed in appended claim 1.

According to a specific embodiment of the present invention, preferably, the catalytic dehydrogenation reaction is carried out in a reaction tube;
a front section of the reaction tube is filled with a catalytic dehydrogenation catalyst, and used to subject propane to the catalytic dehydrogenation reaction;
a rear section of the reaction tube is not filled with a catalyst, and used to subject propane to a steam cracking reaction.

When the temperature of the catalytic dehydrogenation reaction is lower, a portion of the propane is present in the product without being converted, and this portion of propane needs to be separated from the olefin product and recovered in conventional catalytic dehydrogenation reactions of propane. The present invention integrates catalytic dehydrogenation and steam cracking of propane into a single reaction tube by filling the first half of the tube with catalytic dehydrogenation catalyst and the second half of the tube without catalyst, and the integration cannot be realized by existing gas furnaces, and the propane that does not undergo catalytic dehydrogenation can be subjected to steam cracking to obtain products such as methane, ethane, hydrogen, and the like. The separated methane and ethane can be further converted by catalytic conversion to obtain CO, and finally a mixed gas containing hydrogen, CO and the like can be obtained. After adjusting the composition, a syngas suitable for direct reduction of iron can be obtained.

When catalytic dehydrogenation and steam cracking of propane are integrated into a single reaction tube, the water required for steam cracking can be added together with propane or separately added in the middle part of the reaction tube.

According to the present invention, the power is provided by heating the reaction tube (reaction tube for catalytic dehydrogenation reaction, catalytic conversion reaction, steam cracking reaction) by means of an induction coil, and the reaction material inside the reaction tube is supplied with heat from the reaction tube. After the induction coil is supplied with power, electromagnetic induction is generated between the reaction tube and the induction coil, and the reaction tube generates heat, thereby realizing the heating of the reaction material inside the reaction tube. In the process, the induction coil is preferably wrapped around the outside of the reaction tube, and a thermal insulation material (e.g., cement, fireproof material, etc.) can be filled between the reaction tube and the induction coil. Conventional steam cracking devices and catalytic dehydrogenation devices is provided with heat through combustion of fuel oil and gas, and then the reaction tube is heated through heat exchange with the reaction tube to realize the heating of the reaction tube, and thus the cracking and catalytic dehydrogenation raw materials is heated in the reaction tube. However, such heat exchange tends to be not uniform, and the heat would be concentrated in a local area, resulting in that the cracking reaction and the catalytic dehydrogenation reaction is also not uniform. The present invention, on the other hand, heats the reaction tube by means of an induction coil, which has a high heating efficiency, and the induction coil is uniformly distributed on the reaction tube, such that the reaction tube uniformly generates electromagnetic induction, and realizes uniform heating of the cracking raw material and the catalytic dehydrogenation raw material.

According to a specific embodiment of the present invention, preferably, the frequency of the current inputted into the induction coil is medium frequency or high frequency to meet the needs of electromagnetic induction as well as controlling the reaction temperature. During the process of implementation, the frequency of the control current can be selected according to the desired reaction temperature. The high frequency is 5-20 KHz, preferably 8-16 KHz, more preferably 10-15 KHz, further preferably 12-14 KHz, and specifically may be 8 KHz, 8.5 KHz, 9 KHz, 9.5 KHz, 10 KHz, 10.5 KHz, 11 KHz, 11.5 KHz, 12 KHz, 12.5 KHz, 13 KHz, 13.5 KHz, 14 KHz, 14.5 KHz, 15 KHz, 15.5 KHz, 16 KHz, or it may be a range obtained by combining the endpoints of the above ranges as well as the enumerated specific frequency values with each other, such as 5-16 KHz, 5-15 KHz, 5-10 KHz, 8-20 KHz, 8-15 KHz, 8-10 KHz, 10-20 KHz, 10-16 KHz, 10-12 KHz, 9-20 KHz, 9-15 KHz, 12-15 KHz, 12-14 KHz, 12-20 KHz; the medium frequency is 50-3000 Hz, preferably 300-2000 Hz, more preferably 600-1500 Hz, and specifically may be 300 Hz, 400 Hz, 500 Hz, 600 Hz, 700 Hz, 800 Hz, 900 Hz, 1000 Hz, 1100 Hz, 1200 Hz, 1300 Hz, 1400 Hz, 1500 Hz, 1600 Hz, 1700 Hz, 1800 Hz, 1900 Hz, 2000 Hz, or it may be a range obtained by combining the endpoints of the above ranges as well as the enumerated specific frequency values with each other, such as 300-3000 Hz, 300-1500 Hz, 600-3000 Hz, 600-2000 Hz, 1000-3000 Hz, 1000-2000 Hz, 1200-3000 Hz, 1200-2000 Hz, 1500-3000 Hz, 1500-2000 Hz, and the like.

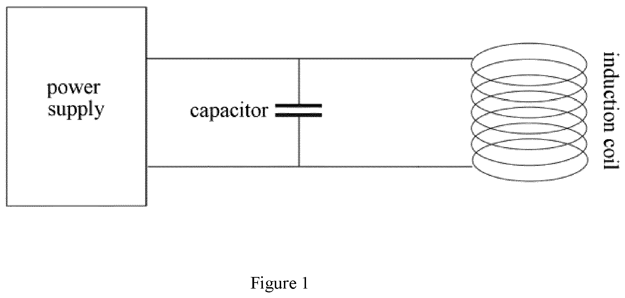

According to a specific embodiment of the present invention, preferably, the frequency of the current inputted into the induction coil is regulated by a power supply and a capacitor. The induction coil is connected to the power supply to form a circuit, and the power supply is connected in parallel with the capacitor as shown in FIG. 1. The power supply used in the present invention may be a commonly used industrial power supply, such as a medium frequency power supply or a high frequency power supply. The power and other specification parameters of the power supply can be selected according to the frequency that needs to be adjusted to, and the rated power of the power supply is preferably 100-1000 KW, and more preferably 200-500 KW. The specification of the capacitor can also be selected as desired, as long as it is sufficient to be able to be matched with the power supply to meet the frequency control requirements.

According to a specific embodiment of the present invention, preferably, the induction coil is one or a combination of two or more selected from ferrite coils, iron core coils, hollow coils, copper core coils and the like.

According to a specific embodiment of the present invention, preferably, the raw material for the catalytic dehydrogenation reaction of propane is propane or a mixed gas of propane and hydrogen.

According to a specific embodiment of the present invention, preferably, in the mixed gas of propane and hydrogen, the volume ratio of propane to hydrogen is from 1:1 to 5:1, preferably 3:1.

According to a specific embodiment of the present invention, preferably, the catalyst for the catalytic dehydrogenation reaction of propane is a platinum-based catalyst or a chromium-based catalyst, more preferably a Pt-Sn-K/Al₂O₃ catalyst or a Cr-K dehydrogenation catalyst.

According to a specific embodiment of the present invention, preferably, the reaction temperature of the catalytic dehydrogenation reaction of propane is 500-1000°C, more preferably 550-850°C, further preferably 550-600°C.

According to a specific embodiment of the present invention, preferably, the reaction temperature of the steam cracking reaction is 500-1000°C, preferably 600-900°C, more preferably 650-850°C, further preferably 750-850°C or 650-750°C.

According to a specific embodiment of the present invention, preferably, the steam cracking reaction has a water-oil ratio of 0.3-0.7, preferably 0.4-0.5.

According to a specific embodiment of the present invention, preferably, the steam cracking reaction has a residence time of 0.1-1.0 s, preferably 0.2-0.85 s.

According to a specific embodiment of the present invention, the separation of the products of the steam cracking reaction can be carried out in a conventional manner, as long as the separation of ethylene, propylene and other gases can be realized. After the separation, a mixed gas containing hydrogen, methane and ethane as well as ethylene and propylene are obtained, wherein the ethylene and propylene can be exported as a product, and the mixed gas is subjected to a further catalytic conversion, such that the methane and propane and water and/or CO₂ undergo a catalytic conversion to obtain CO and H₂, thereby obtaining a syngas, which is transported to the sponge iron production unit for the production of sponge iron.

According to the present invention, the method further comprises the step of adjusting the composition of the syngas to a volume percentage content of CO+H₂ of >90%, and a volume ratio of H₂/CO of 1.5-2.5 (preferably 1.7-1.9), so as to be able to enter into a vertical moving bed reactor for the production of sponge iron. In actual production, if the prepared mixed gas contains sulfur, it can be desulfurized in a conventional process before the catalytic conversion.

According to a specific embodiment of the present invention, preferably, the active component of the catalytic conversion reaction is nickel, and the carrier is one or a combination of two or more selected from alumina, magnesium oxide and magnesium-aluminum spinel; the content of the active component is 5-20% by total mass of the catalyst.

According to a specific embodiment of the present invention, preferably, the reaction conditions of the catalytic conversion reaction are: a pressure of 0.1-1.0 MPa, a reaction temperature of 500-1100°C (preferably 500-850°C), a space velocity of 500-4000 h⁻¹ (preferably 500-2000 h⁻¹), and a volume ratio of water and/or CO₂ to CH₄ of 1.2-1.5/1.

According to a specific embodiment of the present invention, the size of the reaction tube used in the present invention can be selected as desired, wherein the inner diameter of the reaction tube can be 50-250 mm and the length can be selected according to the reaction requirement.

According to a specific embodiment of the present invention, the material of the reaction tube may be a metal or an alloy, respectively, including but not limited to the materials typically used for reaction tubes for steam cracking reaction and reaction tubes for catalytic dehydrogenation reaction. The metal or alloy is preferably one capable of withstanding a temperature of 1000°C, more preferably one capable of withstanding a temperature of 1200°C. The material of the reaction tube of the present invention may be selected from 316L stainless steel, 304S stainless steel, HK40 high-temperature furnace tube material, HP40 high-temperature furnace tube material, HP Micro Alloy micro-alloyed steel, Manaurite XTM material for steam cracking furnace, or the like.

Conventional catalytic dehydrogenation of propane produces propylene and hydrogen, and the hydrogen is emitted as waste and rarely utilized effectively. The present invention combines the olefin industry with iron and steel smelting, realizing the effective utilization of hydrogen and improving the quality of iron and steel smelting at the same time. Moreover, traditional catalysts of the catalytic dehydrogenation of propane deactivate quickly, require periodic regeneration, and require a moving bed process to meet the regeneration and heat absorption requirements of the catalyst, with complex equipment and large investment. By using electric heating for power supply, the present invention allows the use of pipeline fixed-bed dehydrogenation reactors in a series, which are programmed to react, purge and regenerate, and to match the controllable fill of the grid. When grid power consumption is urgently needed, it is possible to have most of the reactors running for dehydrogenation and propylene production, while leaving most of the dehydrogenation reactors in the purge and regeneration state when not needed.

The traditional olefin industry is a high power-consuming industry, and the traditional ethylene industry consumes about 0.5 tons of fuel per ton of ethylene produced. The famous olefin technology companies in the world include Lummus, S&W, KBR, Linde, TPL/KTI, etc. All steam cracking devices are powered by heating with a steam cracking furnace and a fuel burner tube, of which the structure is complicated, the investment in equipment is large, and the investment in cracking furnace accounts for about 30% of the investment in the whole olefin production. In the present invention, it is changed to electric power supply without burner combustion and flue gas power recovery system, and can realize single stove pipe heating and burning carbon processing, as well as stove pipe internal power supply, which is a feature that is difficult to achieve with conventional combustion heating. Furthermore, it is highly innovative and offers a range of degrees of freedom, significantly simplifying the olefin production process and increasing process flexibility. For the production of olefins and hydrogen by using propane, the equipment investment is small, the structure is simple, and power saving and emission reduction can be realized.

In the present invention, based on the fact that propane cannot be fully converted under some reaction conditions, the catalytic dehydrogenation of propane is combined with steam cracking, and the unconverted propane is made into methane, ethane and other components through the steam cracking. Further, by reforming and composition adjustment, a syngas, which is a good raw material for direct reduction of iron, is obtained.

The present invention utilizes electricity to provide power for catalytic dehydrogenation reaction and steam cracking reaction through electromagnetic coil, which is a new use of electricity and solve the current problem of excess electricity. Moreover, utilizing the electromagnetic coil to provide power can make the heat distribution of the reaction tube more uniform, and it is easier to control the reaction temperature and the progress of the reaction.

### Brief Description of the Drawings

FIG. 1 is a schematic circuit diagram of the power supply, electromagnetic coil, and capacitor of the present invention.

### Detailed Description of Preferred Embodiments

In order to have a clearer understanding of the technical features, objectives and beneficial effects of the present invention, the following detailed description of the technical solutions of the present invention is given.

### Example 1

This example provides a steel smelting method comprising the following steps:
the catalyst is filled into a reaction tube, and activated with hydrogen or nitrogen;
the raw materials containing propane are introduced into the reaction tube for a catalytic dehydrogenation reaction;
the products of the catalytic dehydrogenation reaction are separated to give a mixed gas containing hydrogen, methane and ethane, as well as ethylene and propylene in which the ethylene and propylene are output as products;
the mixed gas containing hydrogen, methane and ethane is subjected to the catalytic conversion: the mixed gas is allowed to enter the catalytic conversion reactor tube, and then reacts with the input CO₂ in the catalytic conversion reactor tube to convert hydrocarbons such as methane, ethane, and CO₂ into CO and H₂;
the composition of the catalytically converted gas product is adjusted to the extent that the volume percentage content of CO+H₂ is >90% and the volume ratio of H₂/CO is 1.5-2.5 (preferably 1.7-1.9), and then it is fed into a gas-based shaft furnace for the production of sponge iron;
in the gas-based shaft furnace, iron ore oxide pellets are added from the top of the shaft furnace and move from top to bottom, the syngas enters the furnace from the perimeter pipe of the reduction section at the bottom of the shaft furnace and flows from bottom to top, wherein the syngas undergoes a reduction reaction with the oxide pellets to obtain sponge iron and top gas, with the main reaction: 3H₂+Fe₂O₃=2Fe+3H₂O. There is no carbon dioxide emission during the above process.

The above top gas can be subjected to a washing and cooling treatment, a compression treatment and a desulfurization and decarburization treatment at one time, so as to obtain an unreacted reducing gas.

In the above reaction, electricity is used to provide power for the catalytic dehydrogenation reaction and catalytic conversion reaction through electromagnetic induction, which is carried out using the device shown in FIG. 1. The device includes a power supply (300KW medium-frequency power supply), a capacitor (matching with the medium-frequency power supply), an induction coil (copper-core coil, 30cm in length, wrapped around the outside of the reaction tube), a catalytic dehydrogenation reaction tubes (316L stainless steel, 30 cm in length, 1.7 cm in inner diameter) and a catalytic conversion reaction tube (316L stainless steel, 30 cm in length, 1.7 cm in inner diameter), wherein the induction coil is connected to the power supply to form a circuit, and the power supply is connected in parallel with the capacitor. The power supply is used to adjust the electricity to a current of appropriate frequency, which is then input into the capacitor, through which the induction coil is powered. Electromagnetic induction generated between the reaction tube and the energized induction coil starts generating heat, which heats up the raw materials inside the reaction tube in order to allow the catalytic dehydrogenation and catalytic conversion reactions to take place. In the reaction tube, the raw materials enter from the upper end of the reaction tube and the products leave from the lower end of the reaction tube.

The composition of the propane feedstock used in the example is shown in Table 1.

**Table 1. Composition of the propane feedstock**

| feedsto ck vapor phase | methan e | ethane | ethylen e | propan e | cyclopr opane | propyle ne | iso-but ane | n-butan e | trans -butene | n-buten e | iso-but ene | cis-bute ne |
|---|---|---|---|---|---|---|---|---|---|---|---|---|
| propan e | 0.227 | 0.0595 | 0.0058 | 97.448 5 | 0.0221 | 1.4684 | 0.3638 | 0.1569 | 0.0867 | 0.0889 | 0.0187 | 0.0537 |

The reaction conditions and results are shown in Table 2, in which the catalyst is a Cr-based catalyst commonly used for propane dehydrogenation; the start position for catalyst filling is defined as the position between the top position of the catalyst filled in the reaction tube and the horizontal position of the inductor coil inlet; in the reaction tube, the part of the reaction tubefillwhere the induction coil is wrapped around the outside of the reaction tube is filled with catalyst, starting from the catalyst filling position filland going downward; and in addition to methane, ethane, ethylene, propane, propylene, another component mainly remaining in the product is hydrogen.

The voltage, current and power given in Table 2 are parameters under experimental conditions. **In** industrial applications, the reaction tube would have a larger size, or the like, and the degree of reaction will be different from the experimental conditions. Industrial electricity is generally 220V three-phase or 380V three-phase, and the current and power can be adjusted according to the actual situation (Table 3 shows the upper limit of the parameters under industrial electricity conditions). This difference in parameters does not make a substantial difference to the products.

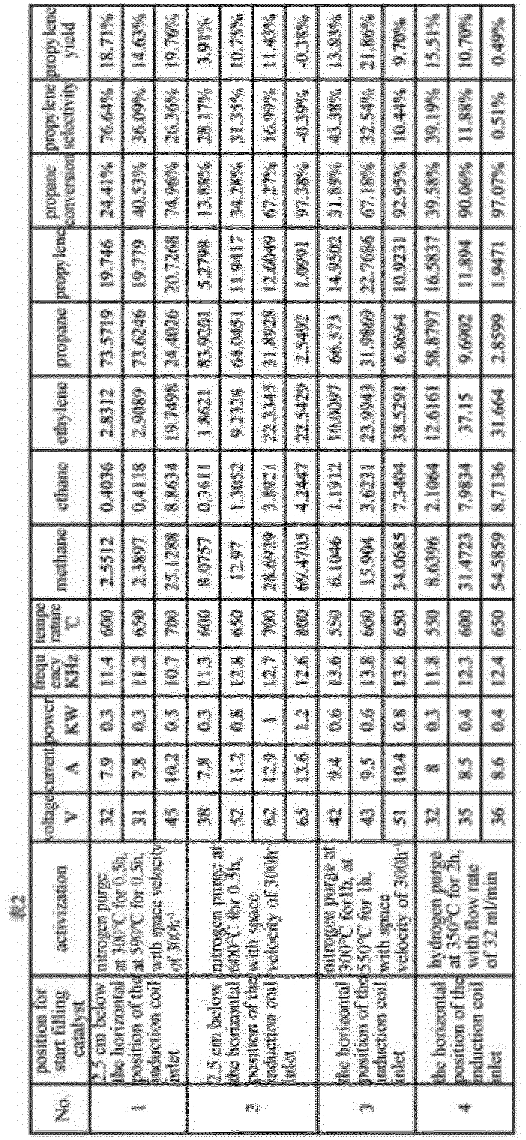

### Example 2

This example provides a steel smelting method in which the catalytic dehydrogenation reaction and the steam cracking reaction of propane are integrated into a single reaction tube, and is the same as Example 1 except for the following steps:
In the reaction tube, catalyst filling begins at a point corresponding to 2.5 cm below the horizontal position of the induction coil inlet; the catalyst is filled until it corresponds to the general length of the induction coil outside the reaction tube, and the remaining portion is not filled with catalyst;
nitrogen is used for activation, with a purge of 0.5 h at 650°C and aspace velocity of 300 h⁻¹;
the raw materials containing propane and water are introduced into the reaction tube for the catalytic dehydrogenation reaction;
the product of the catalytic dehydrogenation reaction undergoes the steam cracking reaction in the lower half of the reaction tube, with the water-oil ratio controlled at 0.4 and a residence time of 0.3 s;
the products of the catalytic dehydrogenation reaction and the steam cracking reaction are separated to give a mixed gas containing hydrogen, methane and ethane, as well as ethylene and propylene, in which the ethylene and propylene are output as products.

Electricity is used to provide power for the steam cracking reaction through electromagnetic coil, with the same equipment, operation and parameters as in Example 1. The reaction conditions and the products are shown in Table 4.

Based on the above, it can be seen that the catalytic dehydrogenation of propane in combination with the production of sponge iron can develop new applications for the by-products of the catalytic dehydrogenation of propane, while providing new applications for electricity.

**Table 3. Parameter upper limits under industrial electricity conditions**

| power | voltage | current | frequency |
|---|---|---|---|
| 200KW | 3-phase 380V | 305A | 5-20KHz |
| 300KW | 3-phase 380V | 455A | 5-20KHz |
| 500KW | 3-phase 380V | 760A | 5-20KHz |
| 200KW | 3-phase 220V | 530A | 5-20KHz |
| 300KW | 3-phase 220V | 790A | 5-20KHz |
| 500KW | 3-phase 220V | 1320A | 5-20KHz |

**Table 4**

| voltage | current | power | frequency | temperature | nethane | ethane | ethylene | propane | propylene | propane conversion | propane selectivity | propylene yield |
|---|---|---|---|---|---|---|---|---|---|---|---|---|
| V | A | KW | KHz | °C | % | % | % | % | % | % | % | % |
| 50 | 10.8 | 0.8 | 13.3 | 650 | 4.3021 | 0.1161 | 0.7072 | 91.3066 | 2.877 | 6.30 | 22.93 | 1.45 |
| 70 | 14 | 1.3 | 13.5 | 700 | 7.8717 | 0.4396 | 4.4786 | 78.4061 | 8.1223 | 19.54 | 34.94 | 6.83 |
| 78 | 15.4 | 1.5 | 13.4 | 800 | 30.8997 | 4.4536 | 20.8537 | 32.5722 | 10.7535 | 66.57 | 14.31 | 9.53 |
| 78 | 15.3 | 1.5 | 13.3 | 850 | 63.2978 | 7.8189 | 24.6991 | 2.3788 | 1.715 | 97.56 | 0.26 | 0.25 |

## Claims

1. A steel smelting method, comprising the steps of:
subjecting propane to a catalytic dehydrogenation reaction, wherein electricity is used to provide power for the catalytic dehydrogenation reaction;
separating the products of the catalytic dehydrogenation reaction to give a mixed gas containing hydrogen, methane, and ethane, as well as ethylene and propylene;
mixing the mixed gas containing hydrogen, methane, and ethane with water and/or CO₂, and then using the mixture as a catalytic conversion feedstock to produce a syngas for sponge iron smelting by a catalytic conversion reaction, wherein electricity is used to provide power for the catalytic conversion reaction;
the method further comprises a step of adjusting the composition of the syngas to a volume percentage content of CO+H₂ of >90%, and a volume ratio of H₂/CO of 1.5-2.5, so as to be able to enter into a vertical moving bed reactor for the production of sponge iron;
the power is provided by heating the reaction tube by means of an induction coil, and the heat is supplied from the reaction tube to the reaction materials inside the reaction tube;
wherein the induction coil is wrapped around the outside of the reaction tube.

2. The method according to claim 1, wherein the catalytic dehydrogenation reaction is carried out in a reaction tube; and
a front section of the reaction tube is filled with a catalytic dehydrogenation catalyst, to allow propane to undergo the catalytic dehydrogenation reaction;
a rear section of the reaction tube is not filled with a catalyst, to allow propane to undergo a steam cracking reaction.

3. The method according to claim 1, wherein the frequency of the current inputted into the induction coil is a medium frequency or a high frequency;
the high frequency is 5-20 KHz;
the medium frequency is 50-3,000 Hz.

4. The method according to claim 1, wherein the frequency of the current inputted into the induction coil is regulated by a power supply and a capacitor;
wherein the induction coil is connected to the power supply to form a circuit, and the power supply is connected in parallel with the capacitor.

5. The method according to claim 4, wherein the power of the power supply is 100-1,000 KW.

6. The method according to claim 1, wherein the induction coil is one or a combination of two or more selected from ferrite coils, iron core coils, hollow coils, and copper core coils.

7. The method according to claim 1, wherein the raw material for the catalytic dehydrogenation reaction is propane or a mixed gas of propane and hydrogen;
the volume ratio of propane to hydrogen is from 1:1 to 5:1.

8. The method according to claim 1, wherein the catalyst for the catalytic dehydrogenation reaction is a Pt-Sn-K/Al₂O; catalyst or a Cr-K dehydrogenation catalyst.

9. The method according to claim 1, wherein the reaction temperature of the catalytic dehydrogenation reaction is 500-1,000°C.

10. The method according to claim 2, wherein the reaction temperature of the steam cracking reaction is 500-1,000°C;
the water-to-oil ratio for the steam cracking reaction is 0.3-0.7, preferably 0.4-0.5; and
the residence time of the steam cracking reaction is 0.1-1.0 s.

11. The method according to claim 1, wherein a catalyst of the catalytic conversion reaction has an active component of nickel, and a carrier which is one or a combination of two or more selected from alumina, magnesium oxide and magnesium-aluminum spinel; and the content of the active component is 5-20% based on the total mass of the catalyst;
the reaction conditions of the catalytic conversion reaction are: a pressure of 0.1-1.0 MPa, a reaction temperature of 500-1,100°C, a space velocity of 500-4,000 h⁻¹, and a volume ratio of water and/or CO₂ to CH₄ of 1.2-1.5/1.

12. The method according to claim 2, wherein the material for the reaction tube is selected from 316L stainless steel, 304S stainless steel, HK40 high-temperature furnace tube material, HP40 high-temperature furnace tube material, HP Micro Alloy micro-alloyed steel or Manaurite XTM material for steam cracking furnace.

13. The method according to claim 2, wherein the reaction tube has an inner diameter of 50-250 mm.

## Patentansprüche

1. Stahlschmelzverfahren, umfassend die folgenden Schritte:
Unterziehen von Propan einer katalytischen Dehydrierungsreaktion, wobei Elektrizität verwendet wird, um Strom für die katalytische Dehydrierungsreaktion bereitzustellen;
Trennen der Produkte der katalytischen Dehydrierungsreaktion, um ein Gasgemisch zu erhalten, das Wasserstoff, Methan und Ethan sowie Ethylen und Propylen enthält;
Mischen des Gasgemisches, das Wasserstoff, Methan und Ethan enthält, mit Wasser und/oder CO₂, und anschließendes Verwenden des Gemisches als Ausgangsmaterial für die katalytische Umwandlung zur Erzeugung von Synthesegas für die Schwammeisenschmelze durch eine katalytische Umwandlungsreaktion, wobei Elektrizität zur Bereitstellung des Stroms für die katalytische Umwandlungsreaktion verwendet wird;
wobei das Verfahren weiter einen Schritt umfasst, bei dem die Zusammensetzung des Synthesegases auf einen Volumenanteil an CO+H₂ von >90 % und ein Volumenverhältnis von H₂/CO von 1,5-2,5 eingestellt wird, damit es in einen vertikalen Fließbettreaktor zur Herstellung von Eisenschwamm eingeleitet werden kann;
wobei der Strom durch Erhitzen des Reaktionsrohrs mittels einer Induktionsspule bereitgestellt wird und die Wärme aus dem Reaktionsrohr an die Reaktionsmaterialien im Inneren des Reaktionsrohrs abgegeben wird;
wobei die Induktionsspule um die Außenseite des Reaktionsrohrs gewickelt ist.

2. Verfahren nach Anspruch 1, wobei die katalytische Dehydrierungsreaktion in einem Reaktionsrohr durchgeführt wird; und
ein vorderer Abschnitt des Reaktionsrohrs mit einem katalytischen Dehydrierungskatalysator gefüllt ist, damit Propan der katalytischen Dehydrierungsreaktion unterzogen werden kann;
ein hinterer Abschnitt des Reaktionsrohrs nicht mit einem Katalysator gefüllt ist, damit Propangas einer Dampfspaltungsreaktion unterzogen werden kann.

3. Verfahren nach Anspruch 1, wobei die Frequenz des in die Induktionsspule eingegebenen Stroms eine mittlere Frequenz oder eine hohe Frequenz ist;
die hohe Frequenz 5-20 kHz beträgt;
die mittlere Frequenz 50-3.000 Hz beträgt.

4. Verfahren nach Anspruch 1, wobei die Frequenz des in die Induktionsspule eingegebenen Stroms durch eine Stromversorgung und einen Kondensator geregelt wird;
wobei die Induktionsspule mit der Stromversorgung verbunden ist, um einen Stromkreis zu bilden, und die Stromversorgung parallel zum Kondensator geschaltet ist.

5. Verfahren nach Anspruch 4, wobei der Strom der Stromversorgung 100-1.000 kW beträgt.

6. Verfahren nach Anspruch 1, wobei die Induktionsspule eine oder eine Kombination aus zwei oder mehr Spulen ist, die aus Ferritspulen, Eisenkernspulen, Hohlspulen und Kupferkernspulen ausgewählt sind.

7. Verfahren nach Anspruch 1, wobei das Rohmaterial für die katalytische Dehydrierungsreaktion Propan oder ein Gemisch aus Propan und Wasserstoff ist;
das Volumenverhältnis von Propan zu Wasserstoff von 1:1 bis 5:1 beträgt.

8. Verfahren nach Anspruch 1, wobei der Katalysator für die katalytische Dehydrierungsreaktion ein Pt-Sn-K/Al₂O₃-Katalysator oder ein Cr-K-Dehydrierungskatalysator ist.

9. Verfahren nach Anspruch 1, wobei die Reaktionstemperatur der katalytischen Dehydrierungsreaktion 500-1.000°C beträgt.

10. Verfahren nach Anspruch 2, wobei die Reaktionstemperatur der Dampfspaltungsreaktion 500-1.000°C beträgt;
das Wasser-Öl-Verhältnis für die Dampfspaltungsreaktion 0,3-0,7, vorzugsweise 0,4-0,5 beträgt; und
die Verweilzeit der Dampfspaltungsreaktion 0,1-1,0 s beträgt.

11. Verfahren nach Anspruch 1, wobei ein Katalysator für die katalytische Umwandlungsreaktion eine aktive Komponente aus Nickel und einen Träger aufweist, der eines oder eine Kombination ist, die aus zwei oder mehr aus Aluminiumoxid, Magnesiumoxid und Magnesium-Aluminium-Spinell ausgewählt ist; und der Gehalt der aktiven Komponente 5-20 % bezogen auf die Gesamtmasse des Katalysators beträgt;
die Reaktionsbedingungen der katalytischen Umwandlungsreaktion wie folgt sind: ein Druck von 0,1-1,0 MPa, eine Reaktionstemperatur von 500-1.100°C, eine Raumgeschwindigkeit von 500-4.000 h⁻¹ und ein Volumenverhältnis von Wasser und/oder CO₂ zu CH₄ von 1,2-1,5/1.

12. Verfahren nach Anspruch 2, wobei das Material für das Reaktionsrohr aus 316L-Edelstahl, 304S-Edelstahl, dem Hochtemperatur-Ofenrohrmaterial HK40, dem Hochtemperatur-Ofenrohrmaterial HP40, dem mikrolegierten Stahl HP Micro Alloy oder dem Material Manaurite XTM für Dampfspaltungsofen.

13. Verfahren nach Anspruch 2, wobei das Reaktionsrohr einen Innendurchmesser von 50-250 mm aufweist.

## Revendications

1. Procédé de fusion d'acier, comprenant les étapes de :
soumission du propane à une réaction de déshydrogénation catalytique, dans lequel de l'électricité est utilisée pour fournir de l'énergie à la réaction de déshydrogénation catalytique ;
séparation des produits de la réaction de déshydrogénation catalytique pour donner un gaz mixte contenant de l'hydrogène, du méthane et de l'éthane, ainsi que de l'éthylène et du propylène ;
mélange du gaz mixte contenant de l'hydrogène, du méthane et de l'éthane avec de l'eau et/ou du CO₂, puis l'utilisation du mélange comme matière première de conversion catalytique pour produire un gaz de synthèse destiné à une fusion de fer spongieux par l'intermédiaire d'une réaction de conversion catalytique, dans lequel de l'électricité est utilisée pour fournir de l'énergie à la réaction de conversion catalytique ;
le procédé comprend en outre une étape d'ajustement de la composition du gaz de synthèse à une teneur en pourcentage volumique de CO+H₂ de >90 %, et un rapport volumique de H₂/CO de 1,5 à 2,5, afin de pouvoir entrer dans un réacteur à lit mobile vertical pour la production de fer spongieux ;
la puissance est fournie en chauffant le tube de réaction par l'intermédiaire d'une bobine d'induction, et la chaleur est fournie du tube de réaction aux matériaux de réaction à l'intérieur du tube de réaction ;
dans lequel la bobine d'induction est enroulée autour de l'extérieur du tube de réaction.

2. Procédé selon la revendication 1, dans lequel la réaction de déshydrogénation catalytique est réalisée dans un tube de réaction ; et
une section avant du tube de réaction est remplie d'un catalyseur de déshydrogénation catalytique, pour permettre au propane de subir la réaction de déshydrogénation catalytique ;
une section arrière du tube de réaction n'est pas remplie d'un catalyseur, afin de permettre au propane de subir une réaction de vapocraquage.

3. Procédé selon la revendication 1, dans lequel la fréquence du courant introduit dans la bobine d'induction est une fréquence moyenne ou une fréquence élevée ;
la haute fréquence est de 5 à 20 kHz ;
la fréquence moyenne est de 50 à 3 000 Hz.

4. Procédé selon la revendication 1, dans lequel la fréquence du courant introduit dans la bobine d'induction est régulée par une alimentation électrique et un condensateur ;
dans lequel la bobine d'induction est connectée à l'alimentation électrique pour former un circuit, et l'alimentation électrique est connectée en parallèle avec le condensateur.

5. Procédé selon la revendication 4, dans lequel la puissance de l'alimentation électrique est de 100 à 1 000 kW.

6. Procédé selon la revendication 1, dans lequel la bobine d'induction est une ou une combinaison de deux ou plus choisies parmi des bobines en ferrite, des bobines à noyau de fer, des bobines creuses et des bobines à noyau de cuivre.

7. Procédé selon la revendication 1, dans lequel la matière première pour la réaction de déshydrogénation catalytique est du propane ou un gaz mixte de propane et d'hydrogène ;
le rapport volumique propane/hydrogène est de 1:1 à 5:1.

8. Procédé selon la revendication 1, dans lequel le catalyseur pour la réaction de déshydrogénation catalytique est un catalyseur de Pt-Sn-K/Al₂O₃ ou un catalyseur de déshydrogénation de Cr-K.

9. Procédé selon la revendication 1, dans lequel la température de réaction de la réaction de déshydrogénation catalytique est de 500 à 1 000°C.

10. Procédé selon la revendication 2, dans lequel la température de réaction de la réaction de vapocraquage est de 500 à 1 000°C ;
le rapport eau/huile pour la réaction de vapocraquage est de 0,3 à 0,7, de préférence de 0,4 à 0,5 ; et
le temps de séjour de la réaction de vapocraquage est de 0,1 à 1,0 s.

11. Procédé selon la revendication 1, dans lequel un catalyseur de la réaction de conversion catalytique présente un composant actif de nickel et un support qui est un ou une combinaison de deux ou plus choisis parmi l'alumine, l'oxyde de magnésium et le spinelle magnésium-aluminium ; et la teneur du composant actif est de 5 à 20 % sur la base de la masse totale du catalyseur ;
les conditions de réaction de la réaction de conversion catalytique sont : une pression de 0,1 à 1,0 MPa, une température de réaction de 500 à 1 100 °C, une vitesse spatiale de 500 à 4 000 h⁻¹et un rapport volumique eau et/ou CO₂ à CH₄ de 1.2 à 1.5/1.

12. Procédé selon la revendication 2, dans lequel le matériau du tube de réaction est choisi parmi acier inoxydable 316L, acier inoxydable 304S, matériau de tube de four à haute température HK40, matériau de tube de four à haute température HP40, acier micro-allié HP Micro Alloy ou matériau Manaurite XTM pour four de vapocraquage.

13. Procédé selon la revendication 2, dans lequel le tube de réaction présente un diamètre intérieur de 50 à 250 mm.
